# EUROPÄISCHE PATENTANMELDUNG

(11) **EP 4 591 779 A1**
(43) Veröffentlichungstag der Anmeldung: **30.07.2025**
(21) Anmeldenummer: 25153234.7
(22) Anmeldetag: 22.01.2025
(51) Int. Cl.: A61B 1/00, A61B 1/04, A61B 5/00, A61B 5/20, A61B 5/01

(54) **BILDGEBUNGSVORRICHTUNG, MEDIZINISCHES SYSTEM UND BILDGEBUNGSVERFAHREN**

(30) Priorität: 24.01.2024 DE 102024101956
(71) Anmelder: Karl Storz SE & Co. KG, 78532 Tuttlingen (DE)
(72) Erfinder: Wenzler, Sebastian, 78532 Tuttlingen (DE); Röschert, Isabelle, 78532 Tuttlingen (DE); Buschle, Lukas, 78532 Tuttlingen (DE)

(57) **Zusammenfassung**

Die vorliegende Erfindung betrifft eine Bildgebungsvorrichtung (10), insbesondere Endoskopvorrichtung (13), umfassend eine Bilderfassungseinheit (12), die dazu eingerichtet ist, multispektrale und/oder hyperspektrale Bilderfassung durchzuführen und Bilddaten eines Objektbereichs (14) zu erzeugen, die räumliche und spektral Information umfassen, und eine Temperaturbestimmungseinheit (16), die dazu eingerichtet ist, nach Maßgabe eines Medienparameters, der ein im Objektbereich vorliegendes Medium anzeigt, und ferner nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht, eine Temperatur des Mediums zu bestimmen.

## Beschreibung

Die vorliegende Anmeldung betrifft eine Bildgebungsvorrichtung, ein medizinisches System und ein Bildgebungsverfahren.

In der medizinischen Diagnostik und Therapie haben sich einige Verfahren etabliert, bei denen Energie auf ein Interventionsgebiet appliziert wird. Ein Beispiel für ein solches Verfahren ist die Lithotripsie. Die Lithotripsie ist ein medizinisches Verfahren zur Behandlung von Nierensteinen oder anderen Organsteinen, bei dem Energiewellen appliziert werden, um die Steine in kleinere Fragmente zu zertrümmern. Die Energiewellen können intrakorporal appliziert werden, beispielsweise bei der Laser- oder Ultraschall-Lithotripsie. Ein weiteres Beispiel ist die Hochfrequenzchirurgie (HF-Chirurgie). Bei der HF-Chirurgie werden hochfrequente elektrische Ströme eingesetzt, um Gewebe präzise zu schneiden und/oder zu koagulieren. So können beispielsweise Schnitte mit minimaler Blutung durchgeführt, Gefäße gezielt verschlossen oder Gewebe verdampft werden. Die HF-Chirurgie wird häufig intrakorporal eingesetzt, beispielsweise bei minimal-invasiven Verfahren wie der Laparoskopie und/oder anderen endoskopischen Verfahren. Bei diesen werden lediglich kleine Inzisionen gesetzt und Instrumente durch diese Inzisionen in eine Körperhöhle eingeführt, um diese zu untersuchen oder einen chirurgischen Eingriff durchzuführen. So lassen sich zum Beispiel die Infektionsgefahr für den Patienten verringern, ästhetische Vorteile erzielen, die postoperativen Schmerzen vermindern und die Blutungsgefahr senken.

Diese Verfahren können in einem Medium wie einer wässrigen Lösung durchgeführt werden. Dies ist etwa in der Urologie der Fall, die sich mit der Prävention, Diagnose und Therapie von Erkrankungen der Nieren und der ableitenden Harnwege befasst. Außerdem werden Eingriffe in der Arthroskopie häufig in einem Medium durchgeführt. Die Arthroskopie ist ein minimal-invasives Verfahren zur diagnostischen und/oder therapeutischen Behandlung von Gelenken. Während des Eingriffs wird häufig ein Medium, beispielsweise eine klare Flüssigkeit wie eine physiologische Kochsalzlösung (0,9 % NaCl), als Spülflüssigkeit verwendet. Diese Spüllösung erfüllt mehrere Funktionen, darunter das Freihalten des Gelenkraums, die Verbesserung der Sicht für den Chirurgen, die Kühlung des Gewebes und die Entfernung von Blut oder anderen Rückständen, um eine klare Sicht auf die zu behandelnden Strukturen zu gewährleisten.

Die Erfinder haben erkannt, dass sich bei einem in einem Medium durchgeführten Verfahren, bei dem Energie appliziert wird, das Medium erwärmen kann. Die Temperatur des Mediums kann derart ansteigen, dass es zu einer lokalen Schädigung des Gewebes kommen kann. Dies kann ein erhöhtes Infektionsrisiko, eine verlängerte Heilungszeit, Narbenbildung und Schmerzen zur Folge haben.

Ausgehend vom Stand der Technik liegt der Erfindung die Aufgabe zugrunde, ein Verfahren, bei dem Energie in einem Medium appliziert wird, sicher durchzuführen.

Die Aufgabe wird erfindungsgemäß durch eine Bildgebungsvorrichtung, insbesondere eine Endoskopvorrichtung, ein medizinisches System und ein Bildgebungsverfahren gelöst, wie sie hierin beschrieben und in den Ansprüchen definiert sind.

Die vorliegende Erfindung sieht vor, eine Bildgebungsvorrichtung, insbesondere Endoskopvorrichtung, bereitzustellen. Die Bildgebungsvorrichtung umfasst eine Bilderfassungseinheit, die dazu eingerichtet ist, multispektrale und/oder hyperspektrale Bilderfassung durchzuführen und Bilddaten eines Objektbereichs zu erzeugen, die räumliche und spektral Information umfassen. Ferner umfasst die Bildgebungsvorrichtung eine Temperaturbestimmungseinheit, die dazu eingerichtet ist, nach Maßgabe eines Medienparameters, der ein im Objektbereich vorliegendes Medium anzeigt, und ferner nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht, eine Temperatur des Mediums zu bestimmen.

Durch die erfindungsgemäßen Merkmale kann ein Verfahren, bei dem Energie in einem Medium appliziert wird, sicher durchgeführt werden. Eine Gefahr einer Gewebeschädigung durch eine erhöhte Temperatur kann reduziert werden. Ferner kann das Verfahren gezielt gesteuert werden und eine zielgenauere Durchführung des Verfahrens erreicht werden. Die Erfinder haben erkannt, dass ein Medium ein charakteristisches Absorptionsverhalten, Reflexionsverhalten und/oder Fluoreszenzverhalten aufweisen kann. Die Erfinder haben ferner erkannt, dass dieses Verhalten gezielt zur Temperaturbestimmung ausgenutzt werden kann. Vorteilhafterweise wird die Temperatur des Mediums anhand der räumlichen und spektralen Information ermittelt. Dadurch ist der Einsatz eines Temperatursensors und/oder dergleichen zur Temperaturbestimmung entbehrlich. Ferner kann, da räumlich aufgelöste Information verwendet werden, auch die Temperaturbestimmung räumlich aufgelöst durchgeführt werden. Räumliche und spektrale Information wird ohnehin bei dem Einsatz der Bildgebungsvorrichtung gewonnen. Aus diesen kann zusätzlich zur Durchführung anderer Analyseverfahren die Temperatur bestimmt werden. Ferner wird die Temperatur spezifisch für unterschiedliche Medien bestimmt. Dadurch kann eine höhere Präzision und Genauigkeit der Temperaturbestimmung erreicht werden.

Die Bildgebungsvorrichtung kann eine mikroskopische, makroskopische und/oder exoskopische Bildgebungsvorrichtung sein. Die Bildgebungsvorrichtung kann als Mikroskop, Makroskop und/oder Exoskop ausgebildet sein und/oder ein solches umfassen. In einigen Ausführungsformen kann die Bildgebungsvorrichtung eine endoskopische Bildgebungsvorrichtung sein. Die Bildgebungsvorrichtung kann eine Endoskopvorrichtung sein. Sie kann ein Endoskop und/oder ein Endoskopsystem umfassen und/oder als ein solches ausgebildet sein und/oder zumindest einen Teil und bevorzugt zumindest einen Großteil und/oder Hauptbestandteil eines Endoskops und/oder eines Endoskopsystems ausbilden. "Zumindest ein Großteil" kann zumindest 55 %, vorzugsweise zumindest 65 %, bevorzugt zumindest 75 %, besonders bevorzugt zumindest 85 % und ganz besonders bevorzugt zumindest 95 % bedeuten, und zwar insbesondere mit Bezug auf ein Volumen und/oder eine Masse eines Objekts.

Die Bildgebungsvorrichtung ist in einigen Ausführungsformen dazu eingerichtet, zur Begutachtung und/oder Beobachtung in einen Hohlraum einführbar zu sein, beispielsweise in eine künstliche und/oder natürliche Kavität, etwa in ein Inneres eines Körpers, in ein Körperorgan wie eine Blase eines Patienten, in eine Gelenkhöhle, in Gewebe oder dergleichen. Die Bildgebungsvorrichtung kann auch dazu eingerichtet sein, zur Begutachtung und/der Beobachtung in ein Gehäuse, eine Verschalung, einen Schacht, ein Rohr oder eine andere, insbesondere künstliche, Struktur einführbar zu sein. Insbesondere kann die Bildgebungsvorrichtung eine medizinische Bildgebungsvorrichtung sein.

Die Bilderfassungseinheit kann eine Optik und/oder eine Bilderfassungssensorik umfassen. Die Bilderfassungseinheit und insbesondere die Optik und/oder die Bilderfassungsensorik kann/können zur multispektralen und/oder hyperspektralen Bildgebung eingerichtet sein, im Speziellen dazu, multispektrale und/oder hyperspektrale Bilddaten zu erfassen und/oder zu erzeugen. Multispektrale Bilderfassung bzw. multispektrale Bilddaten kann sich dabei insbesondere auf solche Bilderfassung beziehen, bei der wenigstens zwei, insbesondere wenigstens drei, und in einigen Fällen wenigstens fünf Spektralbänder voneinander unabhängig erfassbar sind und/oder erfasst werden. Hyperspektrale Bilderfassung bzw. hyperspektrale Bilddaten kann sich dabei insbesondere auf solche Bilderfassung beziehen, bei der wenigstens 20, wenigstens 50 oder sogar wenigstens 100 Spektralbänder voneinander unabhängig erfassbar sind und/oder erfasst werden. Die Bilderfassungseinheit kann nach dem Pushbroom-Verfahren und/oder nach dem Whiskbroom- Verfahren und/oder nach dem Staring- Verfahren und/oder nach einem Schnappschussprinzip arbeiten.

Für einige Anwendungen kann es vorteilhaft sein, eine große spektrale Auflösung verwenden zu können. Es bietet sich dann eine hyperspektrale Bilderfassung an. Für einige Anwendungen kann es vorteilhaft sein, spektrale Bilddaten in Echtzeit zu erzeugen. Spektrale Bilddaten können Bilddaten sein, die räumliche und spektrale Information umfassen. Dies beinhaltet beispielsweise die Erzeugung eines spektral aufgelösten Bilds in weniger als einer Sekunde oder sogar mehrmals pro Sekunde. Hierbei kann es zweckmäßig sein, auf multispektrale Bilderfassung zurückzugreifen. Einer ggf. geringeren spektralen Auflösung steht dann eine höhere Bildwiederholrate gegenüber. Je nach Anwendung kann es hinreichend sein, nur wenige verschiedene Spektralbereiche und/oder Wellenlängen zu berücksichtigen, beispielsweise zwei oder drei oder vier oder generell weniger als zehn. Spektral aufgelöste Bilddaten, die in Echtzeit gewonnen werden bzw. mehrere Bilder pro Sekunde liefern, können auch zu Überwachungszwecken eingesetzt werden, wobei nicht zwingend ein wiederzugebendes Bild für einen Benutzer erstellt werden muss, sondern die Bilddaten auch im Hintergrund verarbeitet werden können.

Die, insbesondere medizinische, Bildgebungsvorrichtung kann wenigstens einen proximalen Abschnitt, einen distalen Abschnitt und/oder einen Zwischenabschnitt aufweisen. Der distale Abschnitt ist insbesondere dazu ausgebildet, in einem Betriebszustand, etwa während einer diagnostischen und/oder therapeutischen Aktion, in eine zu untersuchende Kavität eingeführt zu werden und/oder darin befindlich zu sein. Der proximale Abschnitt ist insbesondere dazu ausgebildet, in einem Betriebszustand, etwa während der diagnostischen und/oder therapeutischen Aktion, außerhalb der zu untersuchenden Kavität angeordnet zu sein. Unter "distal" soll insbesondere bei einer Benutzung einem Patienten zugewandt und/oder einem Benutzer abgewandt verstanden werden. Unter "proximal" soll insbesondere bei einer Benutzung einem Patienten abgewandt und/oder einem Benutzer zugewandt verstanden werden. Insbesondere ist proximal das Gegenteil von distal. Die, insbesondere medizinische, Bildgebungsvorrichtung kann einen Schaft aufweisen, insbesondere einen flexiblen oder starren Schaft. Der Schaft kann ein längliches Objekt sein. Ferner kann der Schaft zumindest teilweise und vorzugsweise zumindest zu einem Großteil den distalen Abschnitt ausbilden. Unter einem "länglichen Objekt" soll insbesondere ein Objekt verstanden werden, dessen Haupterstreckung zumindest um einen Faktor fünf, vorzugsweise zumindest um einen Faktor zehn und besonders bevorzugt zumindest um einen Faktor zwanzig größer ist als eine größte Erstreckung des Objekts senkrecht zu dessen Haupterstreckung, also insbesondere einem Durchmesser des Objekts. Unter einer "Haupterstreckung" eines Objekts, soll insbesondere dessen längste Erstreckung entlang dessen Haupterstreckungsrichtung verstanden werden. Unter einer "Haupterstreckungsrichtung" eines Bauteils soll dabei insbesondere eine Richtung verstanden werden, welche parallel zu einer längsten Kante eines kleinsten gedachten Quaders verläuft, welcher das Bauteil gerade noch vollständig umschließt.

Die Bilderfassungseinheit kann zumindest teilweise und vorzugsweise zumindest zu einem Großteil im Bereich des proximalen Abschnitts angeordnet sein und/oder diesen ausbilden. In anderen Ausführungsformen kann die Bilderfassungseinheit zumindest teilweise und vorzugsweise zumindest zu einem Großteil im distalen Abschnitt angeordnet sein und/oder diesen ausbilden. Ferner kann die Bilderfassungseinheit zumindest teilweise auf den proximalen Abschnitt und den distalen Abschnitt verteilt angeordnet sein. Die Bilderfassungseinheit, insbesondere die Bilderfassungssensorik, weist insbesondere zumindest einen Bildsensor auf. Der Bildsensor kann ein zweidimensionales Pixelmuster aufweisen. Ferner kann die Bilderfassungseinheit auch über zumindest zwei und vorzugsweise mehrere Bildsensoren verfügen, welche hintereinander angeordnet sein können. Ferner können die zwei und vorzugsweise mehreren Bildsensoren über voneinander verschieden ausgebildete spektrale Erfassungsempfindlichkeiten verfügen, sodass beispielsweise ein erster Sensor in einem roten Spektralbereich, ein zweiter Sensor in einem blauen Spektralbereich und ein dritter Sensor in einem grünen Spektralbereich besonders empfindlich bzw. vergleichsweise empfindlicher als die anderen Sensoren ist. Der Bildsensor kann etwa als ein CCD-Sensor und/oder ein CMOS-Sensor ausgebildet sein.

Die Optik der Bilderfassungseinheit kann geeignete optische Elemente wie Linsen, Spiegel, Gitter, Prismen, Lichtwellenleiter etc. umfassen. Die Optik kann dazu eingerichtet sein, von dem Objektbereich kommendes Objektlicht zu der Bilderfassungssensorik zu führen, beispielsweise es zu fokussieren und/oder zu projizieren. Das Objektlicht kann insbesondere von einer Beleuchtung des Objektbereichs herrühren.

Die Bilderfassungseinheit ist insbesondere dazu eingerichtet, zumindest zweidimensionale räumliche Bilddaten zu erzeugen. Die Bilderfassungseinheit kann dahingehend räumlich auflösend sein, dass sie in zumindest zwei unterschiedliche Raumrichtungen jeweils eine Auflösung von zumindest 100 Bildpunkten, vorzugsweise von zumindest 200 Bildpunkten, bevorzugt von zumindest 300 Bildpunkten und vorteilhaft von zumindest 400 Bildpunkten liefert. Die Bilddaten sind vorzugsweise zumindest dreidimensional, wobei zumindest zwei Dimensionen räumliche Dimensionen sind und/oder wobei zumindest eine Dimension eine spektrale Dimension ist. Aus den Bilddaten können mehrere räumlich aufgelöste Bilder des Objektbereichs gewinnbar sein, die jeweils unterschiedlichen Spektralbändern zugeordnet sind. Die räumliche und spektrale Information der Bilddaten kann derart beschaffen sein, dass daraus für mehrere räumliche Bildpunkte jeweils ein zugehöriges Spektrum gewinnbar ist.

In einigen Ausführungsformen ist die Bilderfassungseinheit dazu eingerichtet, laufend aktualisierte Bilddaten zu erzeugen. Die Bilderfassungseinheit kann beispielsweise dazu eingerichtet sein, die Bilddaten im Wesentlichen in Echtzeit zu erzeugen, was beispielsweise eine Erzeugung aktualisierter Bilddaten wenigstens als 30 Sekunden, in einigen Fällen wenigstens als 20 Sekunden und in manchen Fällen sogar wenigstens alle 10 Sekunden oder wenigstens alle 5 Sekunden umfasst.

Der Objektbereich kann wenigstens einen Teil und/oder Abschnitt eines abgebildeten Objekts umfassen. Der Objektbereich kann Gewebe und/oder Organe und/oder ein Teil eines Körpers eines Patienten betreffen. Der Objektbereich kann einen Situs betreffen.

Die Bildgebungsvorrichtung kann eine Beleuchtungseinheit umfassen, die zumindest ein Leuchtmittel umfasst, das dazu eingerichtet ist, in zumindest einem Betriebszustand den Objektbereich zu beleuchten und/oder auszuleuchten. Das Leuchtmittel kann eine Weißlichtquelle, eine, insbesondere durchstimmbare, monochrome Lichtquelle, ein Laser, ein Weißlichtlaser, zumindest eine Leuchtdiode und/oder ein Leuchtdiodenarray, zumindest eine Laserdiode und/oder ein Laserdiodenarray oder dergleichen umfassen. Die Beleuchtungseinheit kann mit der Bilderfassungseinheit integral ausgebildet sein. Insbesondere kann die Beleuchtungseinheit einzelne oder sämtliche Komponenten der Optik der Bilderfassungseinheit nutzen und/oder über eine hiervon separate Beleuchtungsoptik verfügen. Ein Beleuchtungslichtstrahl kann zumindest abschnittsweise koaxial mit einem Messlichtstrahl geführt und/oder führbar sein.

Die Temperaturbestimmungseinheit kann auf einer Recheneinheit implementiert sein. Auf der Recheneinheit können weitere Einheiten, insbesondere Bestimmungseinheiten und/oder Steuereinheiten, implementiert sein.

Der Medienparameter kann ein Parameter sein, der Information über das Medium trägt. Mittels des Medienparameters kann auf das Medium und/oder dessen Beschaffenheit rückschließbar sein. Beispielsweise kann der Medienparameter einen Referenzwert umfassen, der eine Referenzdatenbank wie etwa einen Tabelleneintrag, eine Liste und/oder dergleichen referenziert. Anhand des Referenzwerts kann auf die Referenzdatenbank, insbesondere auf den Tabelleneintrag, die Liste und/oder dergleichen, zurückgegriffen werden. Die Referenzdatenbank, der Tabelleneintrag, die Liste und/oder dergleichen kann vorgespeicherte Parameter betreffend das Medium umfassen. Beispielsweise können spektrale Eigenschaften des Mediums dadurch abrufbar sein. Mittels des Medienparameters kann also vorgespeicherte Information spezifisch für das vorliegende Medium abrufbar sein. Insbesondere kann durch Verwendung des Medienparameters die Temperaturbestimmungseinheit Parameter und/oder Information über das Absorptionsverhalten, das Reflexionsverhalten und/oder das Fluoreszenzverhalten des Mediums erlangen.

Das Medium kann beispielsweise eine elektrolytfreie Spüllösung und/oder eine 0,9-prozentige NaCl-Lösung umfassen.

Das Medium kann insbesondere eine wässrige Lösung, eine Flüssigkeit, ein flüssiges Stoffgemisch und/oder dergleichen sein. Das Medium kann sich während der diagnostischen und/oder therapeutischen Aktion aufwärmen. Das Medium kann ein inhomogenes Temperaturprofil, insbesondere entlang des Objektbereichs, aufweisen. Beispielsweise kann ein Temperaturgradient vorliegen. Das Medium kann eine Körperhöhle zumindest im Wesentlichen ausfüllen, in der die Bildgebungsvorrichtung, insbesondere der Schaft, zumindest teilweise angeordnet ist. Gemäß anderen Ausführungsformen kann das Medium eine Oberfläche einer Struktur, insbesondere anatomischen Struktur, im Objektbereich benetzen und/oder eine Schicht auf der Oberfläche ausbilden. Eine Dicke der Schicht kann wenige Millimeter, etwa 1 mm, 2 mm, oder 5 mm bis zumindest einige Zentimeter, etwa 1 cm, 2 cm, oder 5 cm betragen. Insbesondere muss das Bildgebungsinstrument nicht zwangsläufig in das Medium zumindest teilweise eingetaucht sein. Es kann ausreichend sein, dass das Medium im Objektbereich vorliegt und dass Bilddaten von dem Objektbereich erzeugt werden.

Die Temperatur kann in einem Temperaturbereich zwischen beispielsweise zumindest im Wesentlichen 20 °C und 110 °C, insbesondere 30 °C und 100 °C und/oder 30°C und 45 °C, bestimmbar sein.

Grundsätzlich kann das Medium die Bilddaten, insbesondere die spektrale und räumliche Information, beeinflussen. Beispielsweise kann sich je nach Temperatur des Mediums eine spektrale Verteilung der Bilddaten ändern. Insbesondere kann die spektrale Verteilung in bestimmten Wellenlängenbereichen durch die Temperatur des Mediums veränderbar sein. Die Erfinder haben erkannt, dass die spektrale Verteilung ausgewertet werden kann, um auf die Temperatur des Mediums rückschließen zu können. Vorteilhafterweise kann die Temperaturauflösung ortsaufgelöst bestimmbar sein, da räumliche und spektrale Information erfasst und analysiert wird.

Mit "in den Bilddaten enthaltener räumlicher und spektraler Information, die sich auf ein Absorptionsverhalten, ein Reflexionsverhalten und/oder ein Fluoreszenzverhalten des Mediums beziehen" kann gemeint sein, dass die spektrale Verteilung, insbesondere jedes Pixels, bzw. Bildpunkts, der Bilddaten, durch das Medium in seinem Verlauf beeinflusst werden kann. Insbesondere kann der Verlauf der spektralen Verteilung je nach Temperatur des Mediums für denselben Objektbereich unterschiedlich sein. Je nach Medium kann die spektrale Verteilung unterschiedlich sein. Das kann bedeuten, dass sich Medien bezüglich ihres Absorptionsverhalten, Reflexionsverhaltens und/oder Fluoreszenzverhaltens unterscheiden können.

Grundsätzlich kann sich beispielsweise eine Emissionswellenlänge eines fluoreszierenden Moleküls, das Teil des Mediums sein kann, mit einer Änderung der Temperatur des Mediums spektral verschieben kann. Ein gemessenes Spektrum kann dann beispielsweise eine Verschiebung lokaler Maxima aufweisen im Vergleich zu einem Spektrum desselben Mediums mit einer anderen Temperatur.

Ferner kann sich die Absorption eines Mediums in Abhängigkeit von der Temperatur verändern. Wird das Medium etwa mit Beleuchtungslicht mit denselben Eigenschaften, insbesondere hinsichtlich Intensität und spektraler Verteilung, beleuchtet, kann ein gemessenes Spektrum lokale Maxima und/oder Minima mit unterschiedlich hoher Amplitude aufweisen und/oder diese spektral verschoben sein. In anderen Worten kann das Medium in Abhängigkeit seiner Temperatur Lichtenergie wellenlängenabhängig unterschiedlich absorbieren, bzw. aufnehmen, beispielsweise aufgrund veränderter elektrischer und/ magnetischer Eigenschaften, Resonanzfrequenzen, Phasenübergänge, thermischer Expansion und/oder dergleichen. Dadurch kann eine Reflexion ebenfalls wellenlängenabhängig beeinflusst sein. Das kann bewirken, dass das Medium Licht mit unterschiedlichen Wellenlängen unterschiedlich reflektiert. Allgemein kann Lichtenergie in jeweils einer Wellenlänge anteilig absorbiert und reflektiert werden. Ein Verhältnis von Absorption und Reflexion kann sich wellenlängenspezifisch ändern. Dieses Verhalten kann zudem von der Temperatur des Mediums abhängen. Die Erfinder haben erkannt, dass durch eine Analyse dieses Verhaltens mittels der spektralen Bilddaten die Temperatur des Mediums bestimmbar ist.

Mit Absorption kann grundsätzlich eine Energieaufnahme gemeint sein. Mit Reflexion kann grundsätzlich ein Zurückwerfen einer einfallenden Lichtwelle gemeint sein.

Es versteht sich, dass das Absorptionsverhalten und das Reflexionsverhalten des Mediums eng miteinander verbunden sein können. Sie können sich gegenseitig beeinflussen. Eine höhere Absorption, insbesondere Lichts einer bestimmten Wellenlänge, kann eine niedrigere Reflexion von Licht derselben Wellenlänge bedingen. Insofern kann allgemein von einem Absorptionsverhalten auf ein Reflexionsverhalten rückgeschlossen werden.

Mit der Maßgabe der Information, die sich auf ein Absorptionsverhalten, ein Reflexionsverhalten und/oder ein Fluoreszenzverhalten des Mediums bezieht, kann gemeint sein, dass ein Wellenlängenbereich spezifisch untersucht und/oder analysiert wird. Beispielsweise kann dieser Wellenlängenbereich durch Maßgabe des Medienparameters bestimmbar sein. In anderen Worten kann der zu analysierende Wellenlängenbereich von dem Medium, das im Objektbereich vorliegt, abhängen und durch den Medienparameter bestimmbar sein und/oder bestimmt werden. Beispielsweise kann dieser der Referenzdatenbank entnommen werden. Das Medium kann in einem ersten Wellenlängenbereich eine größere Temperaturabhängigkeit des Absorptionsverhaltens, des Reflexionsverhaltens und/oder des Fluoreszenzverhaltens aufweisen, als in zumindest einem zweiten, insbesondere unterschiedlichen, Wellenlängenbereich.

Die Temperatur kann insbesondere schrittweise bestimmbar sein. Mit schrittweise kann gemeint sein, dass eine Änderung der Temperatur über einen Zeitraum bestimmbar ist. Die Temperatur kann zu verschiedenen Zeitpunkten bestimmt werden und mit der Temperatur eines vorausgegangenen Zeitpunkts verglichen werden. Alternativ oder zusätzlich kann die Temperatur bezogen auf einen Referenzwert bestimmbar sein. Beispielsweise kann ein für ein Medium übliches Absorptionsverhalten, Reflexionsverhalten und/oder Fluoreszenzverhalten der Temperaturbestimmung zugrunde gelegt werden und eine Referenztemperatur angenommen werden. Beispielsweise kann angenommen werden, dass zu einem ersten Zeitpunkt eine bestimmte Temperatur vorliegt. Vorzugsweise ist dieser Zeitpunkt vor einem Zeitpunkt, an dem die diagnostische und/oder therapeutische Aktion vorgenommen wird. Beispielweise wird angenommen, dass die bestimme Temperatur 37 °C und/oder eine andere etwa für die bestimmte Körperhöhle spezifische Temperatur, beträgt. Beispielsweise kann eine Temperatur in einer Gelenkhöhle üblicherweise 36,4 °C betragen. Für diese Temperatur kann ein Referenz-Absorptionsverhalten, -Reflexionsverhalten und/oder -Fluoreszenzverhalten bestimmbar sein, insbesondere mittels der Temperaturbestimmungseinheit. Anhand einer Messung zu einem zweiten Zeitpunkt kann eine Änderung des Absorptionsverhaltens, des Reflexionsverhaltens und/oder des Fluoreszenzverhaltens im Vergleich zu dem Referenz-Absorptionsverhalten, -Reflexionsverhalten und/oder-Fluoreszenzverhalten ermittelt werden und/oder durch die Temperaturbestimmungseinheit ermittelbar sein. Beispielsweise kann ein Vergleichsparameter ermittelbar sein, der auf dem Vergleich des Absorptionsverhaltens, Reflexionsverhaltens und/oder Fluoreszenzverhaltens beruht. Anhand und/oder nach Maßgabe des Vergleichs und/oder des Vergleichsparameters kann die Temperatur bestimmbar sein und/oder bestimmt werden. Grundsätzlich kann sich die schrittweise Temperaturbestimmung auf den Referenzwert beziehen.

Die Temperatur kann mit einer Genauigkeit von bis zu 10°C, 5°C, 2 °C, 1 °C, 0,5 °C, 0,1 °C oder sogar bis zu 0,05 °C bestimmbar sein. Ferner kann die Temperatur mit einer Präzision von bis zu 10°C, 5°C, 2 °C, 1 °C, 0,5 °C, 0,1 °C oder sogar bis zu 0,05 °C. Die Genauigkeit kann von einer Genauigkeit des Referenzwerts abhängen.

Die Bildgebungsvorrichtung lässt sich insbesondere vorteilhaft einsetzen, wenn die Temperaturbestimmungseinheit dazu eingerichtet ist, für unterschiedliche Teilbereiche des Objektbereichs jeweils eine Temperatur des Mediums zu bestimmen. Dadurch können beispielsweise Teilbereiche identifiziert werden, in denen es zu einer lokalen Gewebeschädigung kommen könnte. Ferner kann ein Teilbereich erkannt werden, in dem die diagnostische und/oder therapeutische Aktion durchgeführt wird. Es kann dann etwa abgeschätzt werden, wie sich diese auf benachbarte Teilbereiche auswirkt, insbesondere in Bezug auf die Temperatur des Mediums und/oder im Hinblick auf einer Gewebeschädigung. Die Temperatur des Teilbereichs kann auf mehreren Messwerten beruhen und die Temperatur des Teilbereichs durch einen Mittelwert und/oder dergleichen bestimmt werden. Ferner kann eine notwendige Rechenleistung reduziert werden. Es muss beispielsweise nicht für jeden Bildpunkt eine Temperatur bestimmt werden.

Außerdem kann die Temperaturbestimmungseinheit dazu eingerichtet sein, eine ortsaufgelöste Temperaturverteilung für wenigstens einen Teilbereich des Objektbereichs zu bestimmen. Vorteilhafterweise kann in Teilbereichen von höherer Wichtigkeit eine höhere Temperaturauflösung vorgesehen werden, während in Teilbereichen geringerer Wichtigkeit eine niedrigere Temperaturauflösung vorgesehen wird. Es kann eine verfügbare Rechenleistung gezielt ausgenutzt werden, sodass vorrangig wichtige Information bereitgestellt wird. Beispielsweise kann in zumindest einem Teilbereich um eine Stelle im Objektbereich ortsaufgelöst die Temperaturverteilung bestimmt werden, an der ein Eingriff, etwa eine Gewebeverdampfung, eine Gewebekoagulation und/oder dergleichen, vorgenommen wird. Unter "ortsaufgelöst" kann beispielsweise verstanden werden, dass für jeden Bildpunkt eine Temperatur bestimmt wird. Ferner können auch mehrere Temperaturwerte für benachbarte Bildpunkte gemittelt werden, beispielsweise für 4, 9, 16 und/oder 25 Bildpunkte.

Ferner kann die spektrale Information Intensitätswerte umfassen, die sich auf einen Wellenlängenbereich von, insbesondere vorzugsweise, 700 nm bis 1000 nm beziehen und die Temperaturbestimmungseinheit dazu eingerichtet sein, die Temperatur anhand der Intensitätswerte zu bestimmen, die sich auf den Wellenlängenbereich von 700 nm bis 1000 nm beziehen. Vorteilhafterweise kann ein Absorptionsverhalten, ein Reflexionsverhalten und/oder ein Fluoreszenzverhalten außerhalb des sichtbaren, insbesondere im nahinfraroten Wellenlängenbereich, berücksichtigt werden. Die Erfinder haben erkannt, dass in diesem Wellenlängenbereich das Medium ein stärkeres Absorptionsverhalten, Reflexionsverhalten und/oder Fluoreszenzverhalten aufweisen kann. Ferner kann insbesondere gezielt ein Fluoreszenzverhalten außerhalb des sichtbaren Wellenlängenbereichs ausgenutzt werden. Beispielsweise kann eine elektrolytfreie Spüllösung ein ausgeprägteres temperaturabhängiges Absorptionsverhalten und/oder Reflexionsverhalten zwischen den Wellenlängen von etwa 740 nm und 800 nm als in einem anderen Wellenlängenbereich, insbesondere des sichtbaren Lichts.

Grundsätzlich kann der Wellenlängenbereich auch breiter oder schmaler sein, insbesondere je nach Medium. Ferner kann der Wellenlängenbereich zumindest teilweise außerhalb des Wellenlängenbereichs von 700 nm bis 1000 nm liegen.

Außerdem kann die Temperaturbestimmungseinheit dazu eingerichtet sein, die Temperatur anhand eines Abgleichs der spektralen Information mit einer bekannten Temperaturabhängigkeit eines Absorptionsspektrums, eines Reflexionsspektrums und/oder eines Fluoreszenzspektrums des Mediums zu bestimmen. Dadurch kann eine genaue und/oder fehlerunabhängige Temperaturbestimmung erreicht werden. Der Abgleich kann einen Vergleich des Verlaufs umfassen und/oder einen Vergleich einzelner, insbesondere medienabhängiger, Werte bei vorbestimmten Wellenlängen.

Eine hohe Genauigkeit der Temperaturbestimmung kann erreicht werden, wenn die Temperaturbestimmungseinheit dazu eingerichtet ist, zumindest zwei unterschiedliche Intensitätswerte miteinander zu vergleichen, die sich auf unterschiedliche Spektralbereiche beziehen, um die Temperatur zu bestimmen. Insbesondere können die unterschiedlichen Spektralbereiche innerhalb des Wellenlängenbereichs von 700 nm bis 1000 nm liegen. Die Wahl der Spektralbereich kann insbesondere medienabhängig getroffen werden. Der Medienparameter kann die unterschiedlichen Spektralbereiche anzeigen. In einigen Ausführungsformen werden genau zwei, genau drei und/oder genau vier unterschiedliche Intensitätswerte miteinander verglichen, die sich auf unterschiedliche Spektralbereiche beziehen, um die Temperatur zu bestimmen.

Wiederrum kann eine präzise und akkurate Temperaturbestimmung erreicht werden, wenn die Temperaturbestimmungseinheit dazu eingerichtet ist, die Temperatur anhand wenigstens zweier unterschiedlicher spektraler Stützstellen zu bestimmen, für die das Absorptionsverhalten, Reflexionsverhalten und/oder das Fluoreszenzverhalten des Mediums eine gegenläufige Temperaturabhängigkeit aufweist. Die Erfinder haben erkannt, dass die gegenläufige Temperaturabhängigkeit gezielt ausnutzbar ist. Die Temperatur kann dadurch besser aufgelöst werden und kleinere Temperaturunterschiede erkannt werden. Eine spektrale Stützstelle kann sich beispielsweise auf genau eine Wellenlänge beziehen. Ferner kann eine Stützstelle ein kurzes spektrales Intervall umfassen, wobei das Intervall beispielsweise maximal 10, insbesondere maximal 5, ganzzahlige Wellenlängen breit ist.

Zudem kann die Bilderfassungseinheit dazu eingerichtet sein, in einem Referenzmodus und in einem Beobachtungsmodus betreibbar zu sein, wobei in dem Referenzmodus zumindest ein Referenzbild des Objektbereichs erfassbar ist, bevor dem Objektbereich durch eine Behandlung, insbesondere die therapeutische und/oder diagnostische Aktion, Energie zugeführt wird, wobei das Referenzbild auf räumlich und spektral aufgelösten Bilddaten beruht. Ferner kann in dem Beobachtungsmodus zumindest ein Beobachtungsbild erfassbar sein, während dem Objektbereich durch eine Behandlung Energie zugeführt wird, wobei das Beobachtungsbild auf räumlich und spektral aufgelösten Bilddaten beruht. Zudem kann die Temperaturbestimmungseinheit dazu eingerichtet sein, die Temperatur für das Beobachtungsbild nach Maßgabe des Referenzbilds und des Beobachtungsbilds zu bestimmen. Vorteilhafterweise muss für die Temperaturbestimmung kein eigenes Temperaturbestimmungsbild erzeugt werden. Die Erfinder haben erkannt, dass die Information über die Temperatur bereits in gängigen Beobachtungsbildern vorliegen kann. Ferner haben die Erfinder erkannt, dass diese durch das vorherige Erzeugen des Referenzbilds nutzbar und/oder gewinnbar ist, bzw. dadurch die Temperatur aus Beobachtungsbildern bestimmbar ist. Das Referenzbild kann beispielsweise mit der Referenztemperatur in Verbindung gesetzt werden. Es kann etwa angenommen werden, dass zum Zeitpunkt der Aufnahme des Referenzbilds eine Referenztemperatur vorliegt und das Spektrum der Referenztemperatur zugeordnet werden. Ein Abweichen von der spektralen Verteilung kann dann zur Bestimmung der Temperatur ausgenutzt werden.

Wenn ein "Zeitpunkt" im Zusammenhang mit einer Bilderfassung erwähnt wird, kann sich der "Zeitpunkt" grundsätzlich auf den zur Bilderfassung notwendigen Zeitraum beziehen. Unter dem Zeitpunkt kann also etwa ein Bilderfassungszeitraum verstanden werden.

Ferner kann die Bildgebungsvorrichtung eine Bildanalyseeinheit umfassen, die dazu eingerichtet ist, eine Bilderkennung durchzuführen und nach Maßgabe der Bilderkennung einen Zielbereich des Objektbereichs festzulegen und/oder über eine Bildsequenz zu verfolgen, wobei die Temperaturbestimmungseinheit dazu eingerichtet ist, die Temperatur für den Zielbereich zu bestimmen. Vorteilhafterweise kann zielgerichtet genaue Temperaturinformation eines Bereichs von größerer Wichtigkeit bestimmt werden. Der Zielbereich muss nicht vorausgewählt werden, sondern kann zumindest teilautomatisch bestimmt werden. Der Zielbereich kann dadurch dynamisch verfolgt werden und die Temperaturbestimmung örtlich, dynamisch durchgeführt werden. Insbesondere kann vorgesehen sein, dass in dem Zielbereich die Temperaturbestimmung mit einer größeren Ortsauflösung durchgeführt wird als außerhalb des Zielbereichs. Der Zielbereich kann beispielsweise ein Bereich um eine Energieapplikationsstelle sein. Wenn ein Behandler etwa eine Behandlung flächig durchführt und sich die Energieapplikationsstelle während der Behandlung ändert, kann diese trotzdem verfolgt werden und zielgerichtet wichtige Temperaturinformation mit hoher Ortsauflösung bereitgestellt und/oder bestimmt werden. Die Bildanalyseeinheit kann gemeinsam mit der Temperaturbestimmungseinheit auf der Recheneinheit implementiert sein. Die Bildanalyseeinheit kann etwa auf einer mathematischen Rechenvorschrift umfassend künstliche Intelligenz, Bilderkennung und/oder dergleichen basieren. Die Bildanalyseeinheit kann ein neuronales Netzwerk umfassen und/oder auf maschinellem Lernen beruhen. Beispielsweise kann die Bildanalyseeinheit gezielt für bestimmte Behandlungen trainiert werden.

Außerdem kann die Bilderkennung eine Erkennung eines Behandlungsinstruments umfassen und bei dem Festlegen des Zielbereichs ein Bereich ausgespart werden, in dem sich das Behandlungsinstrument und insbesondere zusätzlich ein definierter Umgebungsbereich um das Behandlungsinstrument herum befindet. Vorteilhafterweise kann dadurch ein Bereich ausgespart werden, in dem es ohnehin zu einer Erhöhung der Temperatur kommen kann, welche auch erwartbar ist. Sofern dieser Bereich jedoch von einem Gewebe beabstandet ist, ist die Temperaturerhöhung nicht zwangsläufig gefährlich. Die Erfinder haben erkannt, dass es von vorrangiger Bedeutung ist, die Temperatur in einem Bereich zu bestimmen, der derart nahe an einem Gewebe und/oder dergleichen ist, dass eine Gefahr für den Patienten entstehen kann. Es kann ein ausgehend vom Behandlungsinstrument bis hin zum Gewebe abfallender Temperaturgradient vorliegen. Ein Behandlungsinstrument kann beispielsweise ein Instrument zur Durchführung von HF-Chirurgie, insbesondere ein bipolares elektrochirurgisches Instrument, sein. Ferner kann das Behandlungsinstrument ein Ultraschallinstrument, ein Röntgeninstrument, ein Laserinstrument und/oder dergleichen sein. Beispielsweise kann das Instrument ein Ultraschall-Skalpell, Laser-Skalpell, ein Elektrokauter, ein Röntgenkatheter, eine photodynamische Therapiefaser, eine Ultraschallsonde, ein Instrument zur Durchführung elektromagnetischer Stoßwellen-Lithotripsie, ein intrakorporales Lichttherapieinstrument und/oder dergleichen umfassen.

Gemäß einigen Ausführungsformen umfasst die Bildgebungsvorrichtung eine Ausgabeeinheit, die dazu eingerichtet ist, dem Benutzer Information bereitzustellen, die sich auf die bestimmte Temperatur bezieht. Vorteilhafterweise kann dem Benutzer, insbesondere visuell, Information betreffend die Temperatur bereitgestellt werden, wodurch der Benutzer beispielsweise eine Gefahr ausgehend von einer gerade durchgeführten Behandlung abschätzen kann. Die Ausgabeeinheit kann etwa ein Monitor, eine Anzeigevorrichtung und/oder dergleichen umfassen. Die Ausgabeeinheit kann beispielsweise eine Temperaturdarstellung erzeugen, die etwa einer spektralen Darstellung und/oder einer Darstellung des Objektbereichs überlagerbar ist. Der Benutzer kann dadurch etwa eine Temperatur einer Stelle des Objektbereichs zuordnen und das Behandlungsinstrument und/oder dergleichen gezielt ansteuern.

Außerdem kann die Bildgebungsvorrichtung eine Steuersignalerzeugungseinheit, die dazu eingerichtet ist, nach Maßgabe der bestimmten Temperatur ein Gerätesteuersignal für ein externes Gerät wie beispielsweise ein Behandlungsinstrument zu erzeugen, und eine Geräteschnittstelle umfassen, an die ein externes Gerät wie beispielsweise ein Behandlungsinstrument anschließbar ist und die dazu eingerichtet ist, das Gerätesteuersignal auszugeben. Vorteilhafterweise kann mittels der bestimmten Temperatur das externe Gerät gesteuert werden und/oder beeinflusst werden. Beispielsweise kann eine Leistung des externen Geräts in Abhängigkeit von der bestimmten Temperatur erhöht und/oder reduziert werden. Die Steuersignalerzeugungseinheit kann gemeinsam mit zumindest der Temperaturbestimmungseinheit auf der Recheneinheit implementiert sein.

Grundsätzlich kann die Recheneinheit durch ein eigenes Gehäuse eingehaust sein.

Insbesondere kann das Gerätesteuersignal ein Notabschaltsignal umfassen. Die Steuersignalerzeugungseinheit kann eine Notabschaltfunktion implementieren. Beispielsweise kann die Steuersignalerzeugungseinheit dazu eingerichtet sein, nach Maßgabe einer kritischen Temperatur, die etwa eine Gewebeschädigung und/oder eine Gefährdung eines/des Patienten bewirken kann, das Notabschaltsignal zu erzeugen. Zudem kann das Notabschaltsignal über die Geräteschnittstelle and das externe Gerät ausgebbar sein. Das Notabschaltsignal kann dazu vorgesehen sein, eine Notabschaltung des externen Geräts zu bewirken. Vorteilhafterweise kann dadurch eine Sicherheitsfunktion implementiert werden, die ein automatisches Abschalten des externen Geräts bewirken kann, sofern eine kritische Temperatur vorliegt. Der Patient kann geschützt werden und etwa eine Energieapplikation mit hoher Sicherheit durchführbar sein.

Ferner sieht die vorliegende Erfindung vor, ein medizinisches System bereitzustellen. Das medizinische System umfasst eine erfindungsgemäße Bildgebungsvorrichtung nach einem Aspekt der Erfindung und ein Gerät, insbesondere ein Behandlungsinstrument, das an eine/die Geräteschnittstelle anschließbar ist und das dazu eingerichtet ist, ein/das Gerätesteuersignal zu verarbeiten. Vorteilhafterweise lassen sich zumindest einige der genannten Vorteile zur Erhöhung der Patientensicherheit ausnutzen. Die Erfinder haben erkannt, dass die erfindungsgemäßen Merkmale vorteilhaft im Zusammenhang mit einem medizinischen System ausgenutzt werden können.

Insbesondere haben die Erfinder erkannt, dass sich ein medizinisches System, das multi- und/oder hyperspektrale Bildgebung implementiert, vorteilhaft mit der erfindungsgemäßen Temperaturbestimmung kombinieren lässt.

Ferner sieht die vorliegende Erfindung vor, ein Bildgebungsverfahren, insbesondere durchgeführt mit einer erfindungsgemäßen Bildgebungsvorrichtung, bereitzustellen. Das Bildgebungsverfahren umfasst den Schritt eines Erfassens von Bildern eines Objektbereichs und Erzeugen multispektraler und/oder hyperspektraler Bilddaten des Objektbereichs, die räumliche und spektral Information umfassen, und den Schritt eines Bestimmens einer Temperatur eines in dem Objektbereich vorliegenden Mediums nach Maßgabe eines Medienparameters, der ein im Objektbereich vorliegendes Medium anzeigt, und ferner nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht.

Das Medium kann eine medizinische Flüssigkeit, insbesondere eine medizinische Spüllösung, sein. Die Temperaturbestimmung kann mit gezielt in Körperhöhlen einbringbaren Flüssigkeiten durchgeführt werden. Medizinische Flüssigkeiten können Lösungen sein, die üblicherweise während Behandlungen zur Verdünnung, Reinigung, Energieübertragung und/oder dergleichen vorgesehen und/oder in eine Körperhöhle eingebracht werden.

Außerdem kann das Medium zumindest ein Additiv, insbesondere einen Farbstoff, enthalten, der in einem Temperaturbereich zwischen 30°C und 100°C ein temperaturabhängiges Absorptionsspektrum, Reflexionsspektrum und/oder Fluoreszenzspektrum aufweist. Es kann ein Medium verwendet werden, dass sich besonders gut für die Temperaturbestimmung eignet. Dadurch kann eine besonders große Genauigkeit und Präzision der Temperaturbestimmung erreicht werden. Das Additiv kann etwa ein Molekül umfassen, das bestimmte Phasenübergänge, elektrische Eigenschaften und/oder dergleichen aufweist, wodurch eine höhere Temperaturabhängigkeit erzielt werden kann. Insbesondere kann durch das Additiv eine gegenläufige Temperaturabhängigkeit des Mediums bei zwei spektralen Stützstellen vorgesehen werden. Ferner kann das Additiv ein Fluoreszenzfarbstoff umfassen. Das kann bedeuten, dass das Medium durch Einbringen des Additivs fluoreszierend machbar ist.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung eines medizinischen Systems und einer Bildgebungsvorrichtung;
- Fig. 2: eine schematische Darstellung eines Objektbereichs;
- Fig. 3: eine weitere schematische Darstellung des Objektbereichs;
- Fig. 4: eine weitere schematische Darstellung des Objektbereichs;
- Fig. 5: eine weitere schematische Darstellung des Objektbereichs;
- Fig. 6: eine schematische Darstellung spektraler Intensitätsverläufe;
- Fig. 7: eine schematische Darstellung spektraler Intensitätsverläufe;
- Fig, 8: eine schematische Darstellung spektraler Intensitätsverläufe;
- Fig. 9: eine schematische Darstellung spektraler Intensitätsverläufe;
- Fig. 10: eine schematische Darstellung einer weiteren Ausführungsform eines medizinischen Systems; und
- Fig. 11: eine schematische Ablaufdiagramm.

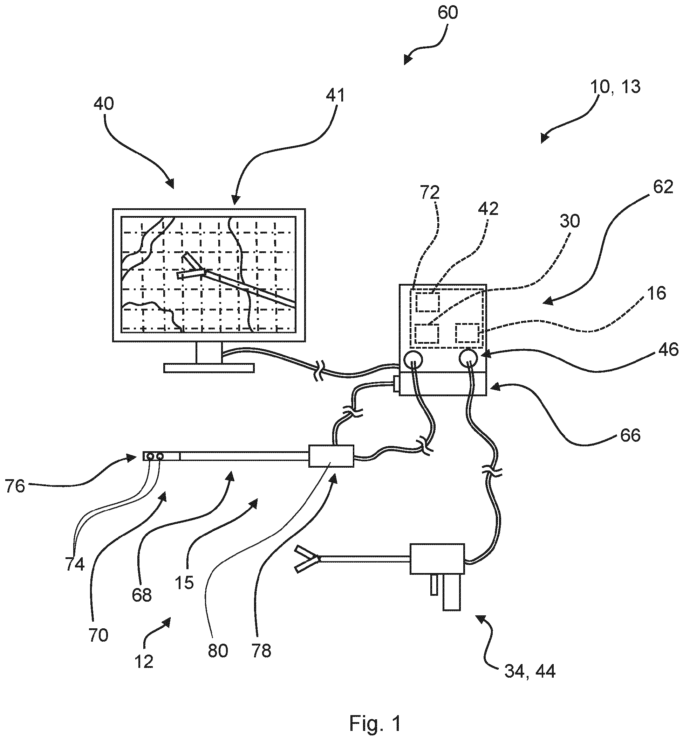

Fig. 1 zeigt eine schematische Darstellung eines medizinischen Systems 60 mit einer medizinischen Bildgebungsvorrichtung 10 und einem Gerät 44. Im exemplarisch dargestellten Fall ist die Bildgebungsvorrichtung 10 eine endoskopische Bildgebungsvorrichtung, konkret eine Endoskopvorrichtung 13, und das Gerät 44 ein Behandlungsinstrument 34, insbesondere ein bipolares elektrochirurgisches Instrument. Andere Instrumente und/oder Geräte, mittels denen Energie auf einen Objektbereich applizierbar ist, können alternativ oder zusätzlich vorgesehen sein.

Alternativ könnte es sich bei der Bildgebungsvorrichtung 10 um eine exoskopische, eine mikroskopische oder eine makroskopische Bildgebungsvorrichtung handeln. Die medizinische Bildgebungsvorrichtung 10 ist zu einer Untersuchung einer Kavität vorgesehen. Mittels des Geräts 44, insbesondere des Behandlungsinstruments 34, ist innerhalb der Kavität Energie auf einen Objektbereich applizierbar.

Das Behandlungsinstruments 34 ist dazu eingerichtet, gezielt Energie in Gewebe einzuleiten, um dieses zu koagulieren, beispielsweise für einen Gefäßverschluss. Diese Ausgestaltung ist rein beispielhaft zu verstehen. Es können sowohl andere Arten von Energieeinleitung vorgesehen sein als auch generell andere Arten medizinischer Vorrichtungen, wie beispielsweise chirurgische, diagnostische, bildgebende, eingriffsunterstützende, anästhetische oder andere medizinische Instrumente und/oder Geräte.

Die Bildgebungsvorrichtung 10 umfasst eine Bilderfassungseinheit 12, beispielhaft umfassend ein handhabbares Instrument 15, insbesondere Endoskop, und ein Bildgebungsgerät 62. Mittels des Bildgebungsgeräts 62 ist das Instrument 15 mit elektrischer Energie versorgbar. Ferner ist das Bildgebungsgerät 62 zur Übermittlung und Empfang von Signalen, insbesondere Bildsignalen, eingerichtet. Dazu umfasst das Bildgebungsgerät 62 die Beleuchtungseinheit 66. Im dargestellten Fall ist die Beleuchtungseinheit 66 über einen Lichtleiter mit dem Instrument 15 verbunden. Beleuchtungslicht ist somit zu der Instrument 15 führbar und von diesem aus auf ein abzubildendes Objekt, insbesondere einen Situs und/oder einen Objektbereich, richtbar.

Das Bildgebungsgerät 62 ist etwa über ein Kabel und/oder eine optische Leitung und/oder einen Lichtleiter mit dem Instrument 15 verbunden.

Die Bildgebungsvorrichtung 10 und insbesondere die Bilderfassungseinheit 12 weist exemplarisch ein oder mehrere Fenster 74 auf, durch die Beleuchtungslicht auskoppelbar und/oder Objektlicht einkoppelbar ist.

Die Bilderfassungseinheit 12, insbesondere das Instrument 15, weist einen distalen Abschnitt 70 auf, der ein distales Ende 76 umfasst. Es handelt sich dabei allgemein ausgedrückt um ein distales Ende 76 der Bildgebungsvorrichtung 10. Der distale Abschnitt 70 ist dazu ausgebildet, in einem Betriebszustand in eine Kavität eingeführt zu werden. Der distale Abschnitt 70 ist in dem Betriebszustand einem Patienten zugewandt. Der distale Abschnitt 70 ist in dem Betriebszustand einem Benutzer abgewandt. Ferner weist die Bilderfassungseinheit 12 einen proximalen Abschnitt 78 auf. Der proximale Abschnitt 78 ist in dem Betriebszustand außerhalb einer Kavität angeordnet. Der proximale Abschnitt 78 ist in dem Betriebszustand dem Patienten abgewandt. Der proximale Abschnitt 78 ist in dem Betriebszustand dem Benutzer zugewandt.

Die Bilderfassungseinheit 12, insbesondere das Instrument 15, weist eine Handhabe 80 auf. Die Handhabe 80 ist exemplarisch zur Handhabung durch den Benutzer eingerichtet. Alternativ oder zusätzlich kann die Handhabe 80 zur Anbringung und/oder Anbindung an einen medizinischen Roboter eingerichtet sein. Die Bilderfassungseinheit 12 kann auch in einigen Ausführungsformen integral mit einem Roboter ausgebildet sein. Eine Position und/oder eine Orientierung der Bilderfassungseinheit 12 relativ zu dem Patienten ist veränderlich, beispielsweise durch Handhabung durch den Benutzer und/oder durch geeignete Bewegung des Roboters.

Das medizinische System 60 weist eine Ausgabeeinheit 40 auf. Exemplarisch ist die Ausgabeeinheit 40 eine Anzeigeeinheit. Die Ausgabeeinheit 40 ist Teil der Bildgebungsvorrichtung 10. Die Ausgabeeinheit 40 kann eine separate Anzeige wie beispielsweise ein Bildschirm oder dergleichen sein und/oder umfassen. In anderen Ausführungsformen kann die Ausgabeeinheit 40 auch in das Bildgebungsgerät 62 integriert sein.

Die Bildgebungsvorrichtung 10 weist eine räumlich und spektral auflösende Bilderfassungseinheit 12 auf, die zumindest eine Optik (nicht dargestellt) aufweist. Die Bilderfassungseinheit 12 weist ferner eine mit der Optik gekoppelte Bilderfassungssensorik (nicht dargestellt), insbesondere einen Bildsensor, der ein zweidimensionales Pixelmuster aufweist, auf. Die Optik und die Bilderfassungssensorik sind dazu eingerichtet, Bilddaten eines Objektbereichs 14 zu erzeugen. Eine Darstellung 41 des Objektbereichs 14 ist in Fig. 1 beispielhaft auf einer Anzeige der Ausgabeeinheit 40 dargestellt. Die Bilddaten umfassen sowohl optische als auch spektrale Information. Die Bilddaten entsprechen im vorliegenden Fall zweidimensionalen räumlichen Daten, die räumliche Bildpunkte definieren, sowie Spektraldaten, die den individuellen Bildpunkten zugeordnet sind. Aus den Bilddaten ist somit für jeden Bildpunkt ein Spektrum erhältlich. Zudem ist aus den Bilddaten für jedes Spektralband ein zweidimensionales Bild erhältlich. Die Bilddaten entsprechen einem multispektralen oder hyperspektralen Datenwürfel.

Die Optik umfasst nicht dargestellte optische Elemente, die Objektlicht sammeln und zur Bilderfassungssensorik führen. Die Bilderfassungssensorik umfasst einen nicht dargestellten CMOS- oder CCD-Sensor. Die Optik und die Bilderfassungssensorik sind gemeinsam in einer Pushbroom-Anordnung angeordnet. In anderen Ausführungsformen wird eine Whiskbroom-Anordnung, eine Staring-Anordnung und/oder eine Schnappschuss-Anordnung verwendet. Die Bilderfassungseinheit 12 ist im vorliegenden Fall zur hyperspektralen Bilderfassung eingerichtet, die Bildgebungsvorrichtung 10 ist entsprechend eine hyperspektrale Bildgebungsvorrichtung. Bezüglich unterschiedlicher Methoden einer hyperspektralen Bildgebung sowie hierfür erforderlicher Komponenten wird auf den Fachartikel "Review of spectral imaging technology in biomedical engineering: achievements and challenges" von Quingli Li et al. Erschienen in Journal of Biomedical Optics 18(10), 100901, Oktober 2013, sowie auf den Fachartikel "Medical hyperspectral imaging: a review" von Guolan Lu und Baowei Fei, erschienen in Journcal of Biomedical Optics 19(1), 010901, Januar 2014, verwiesen. In anderen Ausführungsformen kann die Bildgebungsvorrichtung 10 auch multispektral sein. Mehrere Spektralbereiche können beispielsweise durch wahlweise in einen Objektlichtstrahlengang einbringbare Filter betrachtet werden und/oder durch sequentielle Beleuchtung mit verschiedenen Wellenlängen.

Die Bilderfassungseinheit 12 kann zumindest teilweise in dem Instrument 15 umfasst sein. Teile der Optik und/oder der Bilderfassungssensorik können in dem Bildgebungsgerät 62 umfasst sein. Beispielsweise kann Objektlicht über einen Lichtleiter zu der Bilderfassungssensorik geführt werden und diese in dem Bildgebungsgerät 62 angeordnet sein. In anderen Ausführungsformen ist die gesamte Bilderfassungssensorik in dem Instrument 15 umfasst und es werden lediglich Daten an das Bildgebungsgerät 62 übertragen. Beispielsweise kann die Bilderfassungssensorik innerhalb der Handhabe 80 angeordnet sein.

Die Bildgebungsvorrichtung 10, insbesondere das Bildgebungsgerät 62, umfasst des Weiteren eine Recheneinheit 72 und eine Temperaturbestimmungseinheit 16. Die Temperaturbestimmungseinheit ist auf der Recheneinheit 72 implementiert. Die Temperaturbestimmungseinheit 16 ist dazu eingerichtet, eine Analyse der Bilddaten zu erstellen. Der Analyse liegt sowohl räumliche als auch spektrale Information zugrunde.

Auf der Recheneinheit sind ferner eine Steuersignalerzeugungseinheit 42 und eine Bildanalyseeinheit 30 implementiert.

Gemäß einigen Ausführungsformen umfasst die Bildgebungsvorrichtung 10 zudem eine Fluoreszenzbildgebungseinheit (nicht dargestellt). Die Fluoreszenzbildgebungseinheit ist dazu eingerichtet, den Objektbereich 14 mittels Fluoreszenzbildgebung zu erfassen und Fluoreszenzbildgebungsdaten zu erzeugen. Beispielsweise kann mittels eines Kontrastmittels eine bestimmte Gewebeart durch Fluoreszenzbildgebung erkennbar gemacht werden. Die Fluoreszenzbildgebungsdatenerzeugung mittels der Fluoreszenzbildgebungseinheit stellt einen Modus dar, der möglicherweise zeitgleich, abwechselnd und/oder aufeinanderfolgend mit anderen Modi verwendet werden kann.

In dem vorliegenden Anwendungsbeispiel werden räumlich und spektral aufgelöste Bilddaten nach dem Pushbroom-Verfahren erfasst. Die Temperaturbestimmungseinheit ist nicht auf mittels des Pushbroom-Verfahrens erzeugten Bilddaten beschränkt. Werden beispielsweise Bilddaten nach dem Whiskbroom-Verfahren Punkt für Punkt erfasst, lässt sich die Temperaturbestimmung ebenso anwenden.

Das Instrument 15 umfasst einen Schaft 68, mittels dessen die Bilderfassungseinheit 12 teilweise einer Kavität eines Patienten zuführbar ist. Objektlicht, anhand dessen die multi- und/oder hyperspektralen Bilddaten erzeugbar sind, kann dann etwa durch eines der Fenster 74 in den Schaft 68 einkoppelbar sein. Dieses Licht kann entlang des Schafts 68 zu dem proximalen Abschnitt 78 führbar sein und dort die Bilddaten erzeugbar sein. Derselben Kavität kann das Behandlungsinstrument 34 zugeführt werden, um eine Behandlung wie ein Verschluss eines Gefäßes durchzuführen. Das Behandlungsinstrument 34 ist ein bipolares elektrochirurgisches Instrument. Alternativ kann ein Ultraschall-Skalpell, Laser-Skalpell, ein Elektrokauter, ein Röntgenkatheter, eine photodynamische Therapiefaser, eine Ultraschallsonde, ein Instrument zur Durchführung elektromagnetischer Stoßwellen-Lithotripsie, ein intrakorporales Lichttherapieinstrument und/oder dergleichen verwendet werden.

Die Kavität kann zumindest teilweise mit einem Medium gefüllt sein. Zumindest der distale Abschnitt 70 kann in das Medium getaucht sein, wie zudem auch ein Abschnitt, insbesondere ein distaler Abschnitt, des Behandlungsinstruments 34. Insbesondere ist der Objektbereich 14 mit dem Medium bedeckt.

Die Erfinder haben erkannt, dass sich das Medium bei einer Benutzung des Behandlungsinstruments 34 in der Kavität erwärmen kann, etwa während der Durchführung einer Behandlung wie dem Verschluss des Gefäßes. Dadurch kann es vorkommen, dass es zu einer lokalen Gewebeschädigung innerhalb des Objektbereichs kommen.

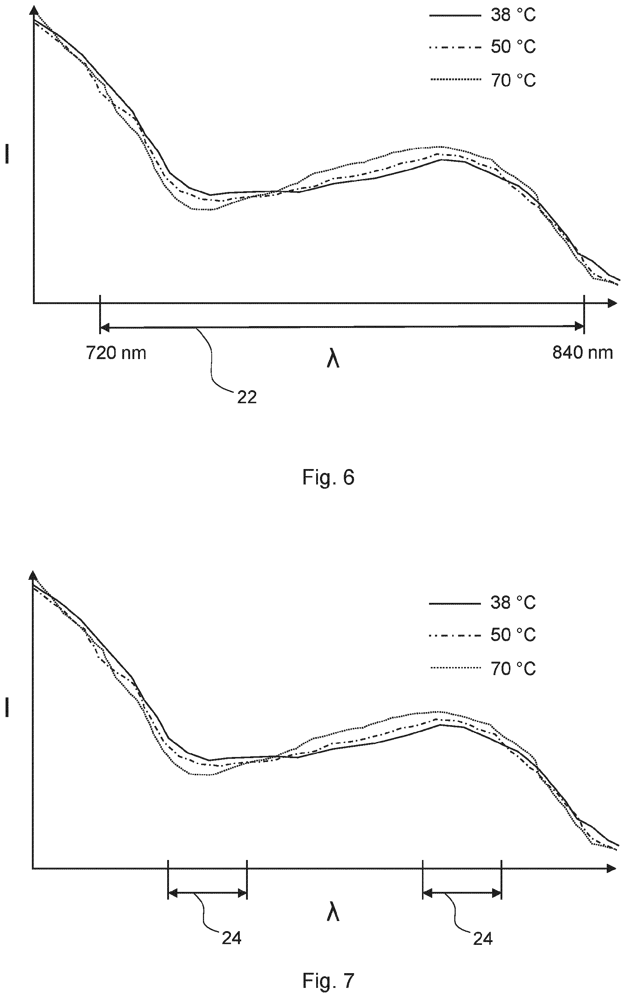

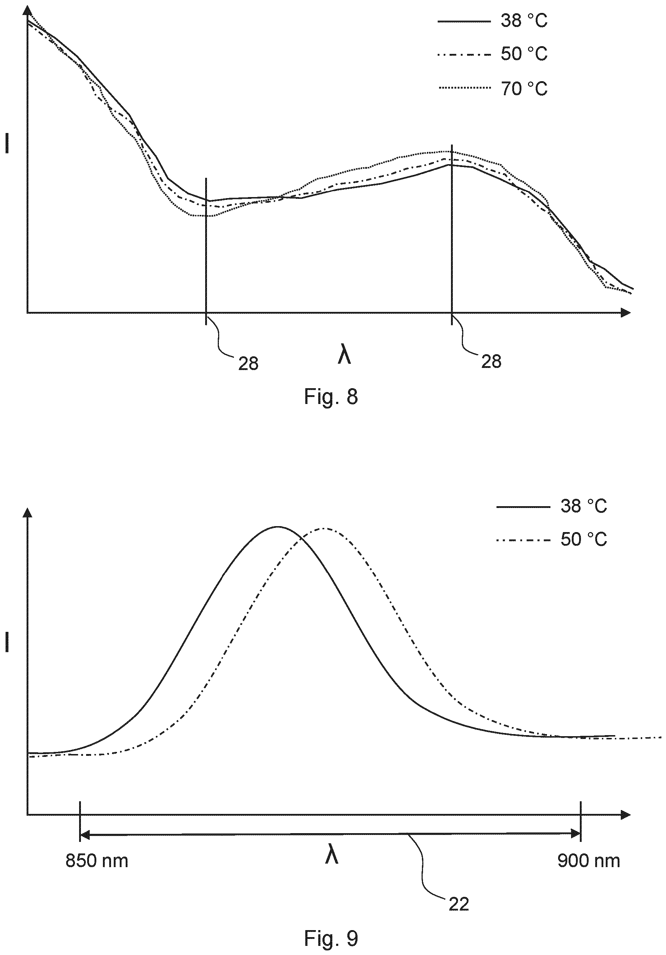

Die Erfinder haben jedoch erkannt, dass sich in Abhängigkeit von der Temperatur ein Absorptionsverhalten, ein Reflexionsverhalten und/oder ein Fluoreszenzverhalten des Mediums ändern kann. Beispielsweise kann sich, wie in den Fig. 6 bis 9 gezeigt ist, ein Verlauf eines Spektrums an einem Bildpunkt eines mittels der Bilderfassungseinheit 12 erfassten Hyperspektralbilds verändern. Die Temperaturabhängigkeit kann für verschiedene Medium unterschiedlich sein. Beispielhaft wird im Folgenden eine Temperaturabhängigkeit einer elektrolytfreien Spüllösung wie etwa "Purisole SM" beschrieben. Anderen Lösungen und/oder Medien können eine andere Abhängigkeit aufweisen. Insbesondere wird eine Temperaturabhängigkeit eines Fluorophors in Fig. 9 beispielhaft gezeigt.

In einigen Ausführungsformen ist das Medium eine medizinische Flüssigkeit, insbesondere eine medizinische Spüllösung. Diese wird beispielsweise zur Reinigung der Kavität und/oder des Instruments verwendet. Ferner kann in einigen Ausführungsformen das Medium zumindest ein Additiv, insbesondere einen Farbstoff, enthalten. Das Additiv weist etwa in einem Temperaturbereich zwischen 30°C und 100°C ein temperaturabhängiges Absorptionsspektrum, Reflexionsspektrum und/oder Fluoreszenzspektrum auf. Dadurch kann gezielt das Absorptionsspektrum, Reflexionsspektrum und/oder Fluoreszenzspektrum eingestellt werden.

Um für ein spezifisches Medium die Temperatur zu bestimmen, wird der Temperaturbestimmungseinheit 16 ein Medienparameter beispielsweise mitgeteilt. Dies kann ein Benutzer etwa über eine nicht dargestellt Benutzerschnittstelle durchführen. Exemplarisch kann der Benutzer ein vordefiniertes Medium aus einer Listenauswahl auswählen, wodurch eine Maßgabe des Medienparameters durch die Temperaturbestimmungseinheit 16 bewirkt wird. Alternativ oder zusätzlich kann ein Medium erkannt werden. Mittels des Medienparameters kann die Temperaturbestimmungseinheit 16 etwa medienspezifische spektrale Parameter, spektrale Verläufe, Information über eine Temperaturabhängigkeit und/oder dergleichen erhalten.

Nach Maßgabe des Medienparameters bestimmt die Temperaturbestimmungseinheit 16 anhand von in den Bilddaten enthaltener räumlicher und spektraler Information, die sich auf ein Absorptionsverhalten, ein Reflexionsverhalten und/oder ein Fluoreszenzverhalten des Mediums bezieht, eine Temperatur des Mediums. Genauer bestimmt die Temperaturbestimmungseinheit 16 nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht, eine Temperatur des Mediums. Es können also Bilddaten für die Temperaturbestimmung verwendet werden, die im Zuge einer gewöhnlichen multi- und/oder hyperspektralen Bilderfassung erzeugt wurden. Die Temperaturbestimmung kann also parallel zu einem anderen Verfahren und/oder zur Bildgebung durchgeführt werden. Alternativ kann auch gezielt eine multi- und/oder hyperspektrale Bildgebung für die Temperaturbestimmung durchgeführt werden.

Die Temperaturbestimmungseinheit 16 analysiert beispielsweise einen Verlauf des Spektrums an einem und/oder mehreren Bildpunkten, um auf die Temperatur rückzuschließen. Dies geschieht unter Berücksichtigung des Mediums und/oder des Medienparameters.

Die Steuersignalerzeugungseinheit 42 ist dazu eingerichtet, nach Maßgabe der bestimmten Temperatur ein Gerätesteuersignal für das Gerät 44, insbesondere das Behandlungsinstrument 34, zu erzeugen. Die Geräteschnittstelle 46 ist dazu eingerichtet, das Gerätesteuersignal auszugeben. Das Gerät 44, insbesondere das Behandlungsinstrument 34, das an die Geräteschnittstelle 46 anschließbar ist und angeschlossen ist, ist dazu eingerichtet, das Gerätesteuersignal zu verarbeiten. Dadurch kann eine Notabschaltfunktion implementiert werden. Wird etwa eine Temperatur über einem Grenzwert bestimmt, kann ein Geräteabschaltsignal durch die Geräteschnittstelle 46 ausgegeben werden. Ferner kann etwa mittels des Gerätesteuersignals eine Leistung des Geräts 44 gesteuert und sogar geregelt werden. Die Funktion des Geräts kann derart gesteuert und/oder geregelt werden, dass eine des Mediums unter einem Grenzwert wie etwa 41 °C bleibt.

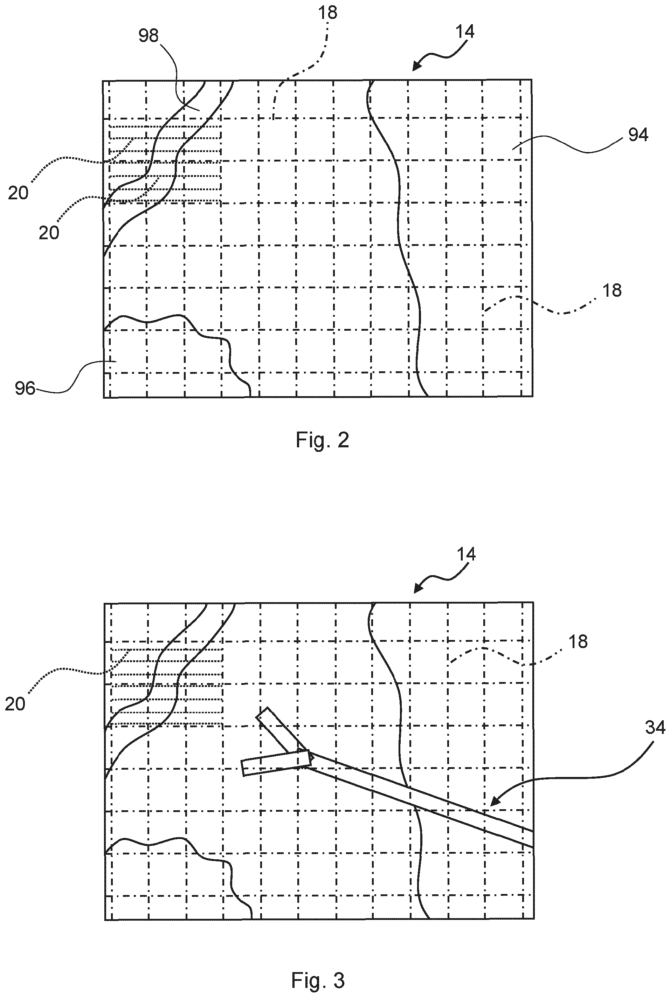

Fig. 2 zeigt eine schematische Darstellung eines Objektbereichs 14. Der Objektbereich 14 wird etwa im Rahmen eines mikroinvasiven Eingriffs beobachtet. Hierfür ist der distale Abschnitt 70 des Instruments 15 (vgl. Fig. 1) in eine Kavität eines Patienten eingeführt. Andere Kavitäten sind ebenso denkbar, insbesondere auch im nicht-medizinischen Bereich. Im Objektbereich 14 befinden sich verschiedene native Strukturen 94, 96 sowie ein Gefäß 98, beispielsweise ein Blutgefäß, das mittels des Behandlungsinstruments 34 versiegelt werden soll.

Zunächst werden räumlich und spektral aufgelöste Bilddaten des Objektbereichs 14 erzeugt. Hierfür wird der Objektbereich 14 beleuchtet und es wird daraufhin vom Objektbereich 14 kommendes Objektlicht erfasst. Die erfasste spektrale Information betrifft im vorliegenden Fall Lichtabsorption. Für jeden Bildpunkt kann entsprechend ein Intensitätsspektrum erhalten werden. Durch veränderte, temperaturabhängige Absorption kann sich das Reflexionsverhalten ändern, wodurch das Intensitätsspektrum beeinflussbar ist.

Die Temperaturbestimmungseinheit 16 ist dazu eingerichtet, für unterschiedliche Teilbereiche 18 des Objektbereichs 14 jeweils eine Temperatur des Mediums zu bestimmen. Der zumindest im Wesentlichen gesamte Objektbereich 14 kann etwa in Teilbereiche 18 unterteilt werden. Für jeden der in der Fig. 2 mit der Strichpunktlinie gekennzeichneten Teilbereiche 18 kann eine Temperatur bestimmt werden, beispielsweise durch Mittelung von Temperaturen mehrerer Bildpunkte eines Teilbereichs 18. Alternativ kann auch lediglich eine Temperatur für einen repräsentativen Bildpunkt bestimmt werden. An der Ausgabeeinheit kann dann etwa eine Darstellung 41 des Objektbereichs 14 erzeugbar sein (vgl. Fig. 1). Der Darstellung 41 kann eine Temperaturdarstellung überlagerbar sein, die für jeden der Teilbereiche 18 eine Temperatur anzeigt. Dies kann etwa in Form einer Heatmap und/oder dergleichen durchgeführt werden, wobei jeder Temperatur eine unterschiedliche Farbe und/oder ein unterschiedlicher Farbton zuordenbar ist. Die Temperaturdarstellung kann der Darstellung 41 überlagert dargestellt sein. Ein Benutzer kann dann jedem Teilbereich 18 des Objektbereichs 14 eine Temperatur zuordnen. Eine Größe der Teilbereiche 18 ist exemplarisch zu verstehen. Sie können relativ zum Objektbereich größer und/oder kleiner gewählt werden.

Ferner erkennt man in der Fig. 2 zusätzlich durch Punktlinien gekennzeichnete Teilbereiche 20, die einer Auswahl an Teilbereichen 18 entsprechen können. Die Temperaturbestimmungseinheit 16 ist dazu eingerichtet, eine ortsaufgelöste Temperaturverteilung für die Teilbereiche 20 des Objektbereichs 14 zu bestimmen. In diesen Teilbereichen 20 wird die Temperatur für jeden Bildpunkt bestimmt. Dies kann ebenfalls in der Temperaturdarstellung dargestellt werden. Der Benutzer kann dadurch eine für diese Teilbereiche detaillierte Temperaturinformation erhalten.

Gemäß einer Ausführungsform bestimmt die Temperaturbestimmungseinheit 16 die Temperatur anhand eines Abgleichs der spektralen Information mit einer bekannten Temperaturabhängigkeit eines Absorptionsspektrums und/oder eines Reflexionsspektrums des Mediums. Die Information über die Temperaturabhängigkeit erhält die Temperaturbestimmungseinheit 16 durch die Maßgabe des Medienparameters. In einer Datenbank (nicht dargestellt) sind derartige Temperaturabhängigkeiten, Absorptionsspektra und/oder Reflexionsspektra hinterlegt.

Exemplarisch umfasst die spektrale Information Intensitätswerte, die sich auf einen Wellenlängenbereich von 700 nm bis 1000 nm beziehen. Die Temperaturbestimmungseinheit 16 ist dazu eingerichtet, die Temperatur anhand der Intensitätswerte zu bestimmen, die sich auf den Wellenlängenbereich von 700 nm bis 1000 nm beziehen.

Alternativ oder zusätzlich ist die Bilderfassungseinheit 12 dazu eingerichtet, in einem Referenzmodus und in einem Beobachtungsmodus betreibbar zu sein. In dem Referenzmodus ist zumindest ein Referenzbild des Objektbereichs 14 erfassbar. Das Referenzbild wird erfasst, bevor dem Objektbereich 14 durch eine Behandlung Energie zugeführt wird. Das Referenzbild beruht auf räumlich und spektral aufgelösten Bilddaten. Das Referenzbild kann etwa entsprechend den Ausführungen im Zusammenhang mit der Fig. 2 entsprechen. Der in der Fig. 2 gezeigte Objektbereich 14 wird also erfasst, bevor dem Objektbereich 14 Energie zugeführt wird. Für jeden Teilbereich 18, Teilbereich 20 und/oder jeden Bildpunkt kann ein Referenzspektrum erzeugbar sein. Dieses wird zur späteren Durchführung eines Abgleichs hinterlegt, bzw. gespeichert. In dem Beobachtungsmodus ist zumindest ein Beobachtungsbild erfassbar, während dem Objektbereich 14 durch eine Behandlung Energie zugeführt wird, wobei das Beobachtungsbild auf räumlich und spektral aufgelösten Bilddaten beruht.

Der entsprechende Objektbereich 14 ist schematisch in der Fig. 3 gezeigt. Man erkennt grundsätzlich denselben Objektbereich 14 wie in der Fig. 2. Zusätzlich erkennt man das Behandlungsinstrument 34, das der Benutzer ebenfalls der Kavität zugeführt hat. Die Temperaturbestimmungseinheit 16 ist dazu eingerichtet, die Temperatur für das Beobachtungsbild nach Maßgabe des Referenzbilds und des Beobachtungsbilds zu bestimmen. Dazu gleicht die Temperaturbestimmungseinheit beispielsweise die entsprechenden Spektra beider Bilder ab. Anhand einer Veränderung der Spektra kann zumindest auf eine Veränderung der Temperatur des Mediums zwischen den Zeitpunkten der Bilderfassung der Bilder geschlossen werden. Ferner kann etwa angenommen werden, dass zum Zeitpunkt der Referenzbilderfassung eine Körpertemperatur von 37 °C vorlag. Anhand dieser Referenztemperatur kann die Temperatur zum Zeitpunkt der Beobachtungsbilderfassung bestimmt werden, insbesondere für jeden Teilbereich 18, 20 und/oder jeden Bildpunkt. Weitere Vorgehensweisen zur Bestimmung der Temperatur werden im Zusammenhang mit den Fig. 6 bis 8 beschrieben.

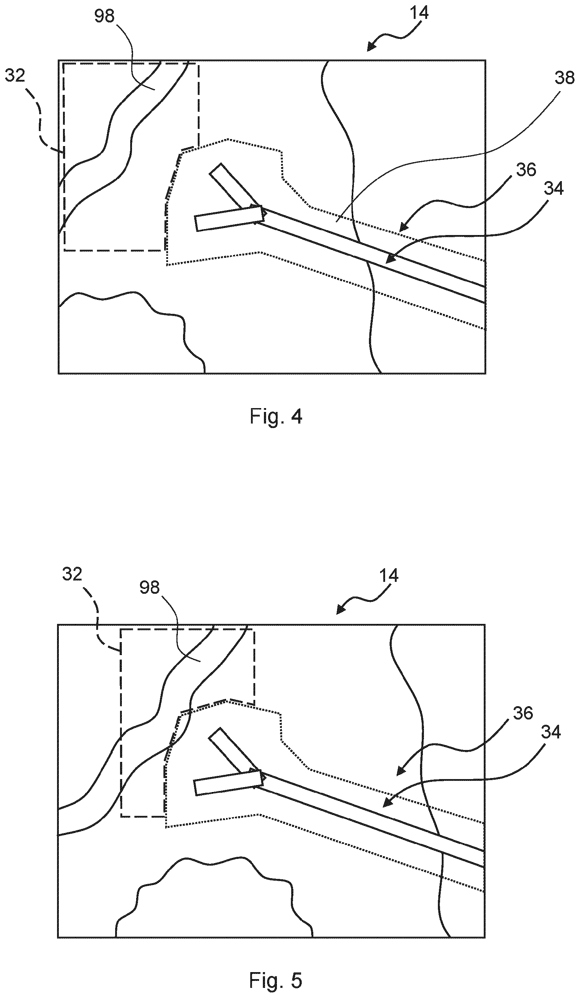

Die Fig. 4 zeigt abermals eine schematische Darstellung des Objektbereichs 14 mit dem zu behandelnden Gefäß 98. Die Bildanalyseeinheit 30 (vgl. Fig. 1) ist dazu eingerichtet, eine Bilderkennung durchzuführen. Nach Maßgabe der Bilderkennung legt die Bildanalyseeinheit 30 einen Zielbereich 32 des Objektbereichs 14 fest. Gemäß der Fig. 4 erkennt die Bildanalyseeinheit das Gefäß 98 und legt einen Bereich um das Gefäß als Zielbereich 32 fest. Allgemein kann die Bildanalyseeinheit 30 zur Erkennung bestimmter Strukturen und/oder Merkmale eingerichtet sein. Beispielsweise kann die Bildanalyseeinheit 30 basierend auf einem neuronalen Netz die Strukturen und/oder Merkmale erkennen. Der Benutzer kann der Bildanalyseeinheit 30 vorgeben, welche Struktur sie erkennen soll, beispielsweise je nach durchzuführender Behandlung. Die Temperaturbestimmungseinheit 16 ist dazu eingerichtet, die Temperatur für den Zielbereich 32 zu bestimmen. Der Benutzer kann also die zu behandelnde und/oder eine sensible Struktur auswählen, um die der Zielbereich 32 festgelegt wird. Durch die Ausgabeeinheit 40 kann der Benutzer dann Information über die Temperatur in dem Zielbereich erhalten.

Ferner umfasst die Bilderkennung eine Erkennung des Behandlungsinstruments 34. Die Bildanalyseeinheit 30 legt einen Bereich 36 um das Behandlungsinstrument 34 herum fest, wobei der Bereich 36 einen Umgebungsbereich 38 um das Behandlungsinstrument 34 herum umfasst. Bei dem Festlegen des Zielbereichs 32 wird der Bereich 36 ausgespart. Soll, wie exemplarisch in der Fig. 4 gezeigt, ein rechteckiger Zielbereich 32 festgelegt werden, wird dieser durch den Bereich 36 beschnitten.

Außerdem ist die Bildanalyseeinheit 30 dazu eingerichtet, den Zielbereich 32 über eine Bildsequenz zu verfolgen. In der Fig. 5 ist exemplarisch eine schematische Darstellung des Objektbereichs 14 zum einem im Vergleich zur Fig. 4 späteren Zeitpunkt einer Bildsequenz gezeigt. Man erkennt, dass der Objektbereich 14 leicht verschoben ist. Eine Bilderfassung dieses Objektbereichs 14 wird durchgeführt. Eine Verschiebung des Objektbereichs 14 kann dadurch vorkommen, dass die Bilderfassungseinheit 12 (vgl. Fig. 1) bezüglich des Objektbereichs 14 bewegt wird. Die Bildanalyseeinheit 30 verfolgt das Gefäß 98 und/oder den Zielbereich 32 von Bild zu Bild der Bildsequenz. Der Zielbereich 32 ist daher zumindest im Wesentlichen an derselben Stelle relativ zum Objektbereich angeordnet im Vergleich mit dem Zielbereich 32 der Fig. 4. Zusätzlich erkennt man, dass das Behandlungsinstrument 34 bewegt wurde. Dadurch hat sich auch der Bereich 36 bewegt. Bei der Festlegung des Zielbereichs 32 wird daher ein größerer Abschnitt im Vergleich zu der Fig. 4 von der Festlegung des Zielbereichs 32 ausgespart.

In den Fig. 6 bis 9 wird eine Temperaturabhängigkeit des Mediums anhand spektraler Verläufe schematisch dargestellt. An der Abszisse sind jeweils Wellenlängen von einer kleineren hin zu einer größeren Wellenlänge aufgezeichnet. An der Ordinate sind gemessene Intensitätswerte an einem Bildpunkt aufgezeichnet. In den Fig. 6 bis 8 erkennt man drei spektrale Verläufe, die jeweils mit einer unterschiedlichen Linie gekennzeichnet sind. Den Verläufen liegt ein temperaturabhängiges Absorptionsverhalten zugrunde. Durch die Temperaturabhängigkeit absorbiert das Medium bei derselben Wellenlänge je nach Temperatur einfallendem Licht in einem unterschiedlichen Ausmaß Energie, wodurch reflektiertes abhängig von der Medientemperatur unterschiedliche Intensitätsverläufe aufweist. Die durchgezogene Linie entspricht einem spektralen Verlauf bei einer Medientemperatur von 38 °C, die Strichpunktlinie einem Verlauf bei 50 °C und die Punktlinie einem Verlauf bei 70 °C. Die Temperaturabhängigkeit für das spezifische Medium liegt in einem Wellenlängenbereich 22 zwischen 700 nm und 1000 nm, genauer zwischen 720 nm und 840 nm, vor. Anhand der Intensitätswerte, die an der Ordinate aufgezeichnet sind, kann die Temperaturbestimmungseinheit 16 die Temperatur bestimmen. Beispielsweise indem ein gesamter Verlauf in dem Wellenlängenbereich 22 verglichen wird. Die Temperatur kann also anhand der Abweichung des spektralen Verlaufs in Abhängigkeit von der Temperatur bestimmt werden. Ferner kann ein Verlauf, wie er in der Fig. 6 exemplarisch gezeigt ist, mit einem vorgespeicherten, bekannten Verlauf verglichen werden.

Man erkennt in der Fig. 6 zudem, dass das Absorptionsverhalten und/oder das Reflexionsverhalten des Mediums eine gegenläufige Temperaturabhängigkeit aufweist. In einem Bereich näher bei einer Wellenlänge von 720 nm sind Intensitätswerte bei einer niedrigeren Medientemperatur (durchgezogene Linie) höher relativ zu einer höheren Medientemperatur. In einem Bereich näher bei einer Wellenlänge von 840 nm sind die Intensitätswerte hingegen niedriger bei einer niedrigeren Medientemperatur. Die Erfinder haben erkannt, dass dieses Verhalten gezielt ausgenutzt werden kann.

Beispielsweise kann, wie in der Fig. 7 schematisch dargestellt, die Temperaturbestimmungseinheit 16 dazu eingerichtet sein, zumindest zwei unterschiedliche Intensitätswerte miteinander zu vergleichen, die sich auf unterschiedliche Spektralbereiche 24 beziehen, um die Temperatur zu bestimmen. Die Spektralbereiche 24 können derart gewählt sein, dass das Medium eine gegenläufige Temperaturabhängigkeit in den Spektralbereichen 24 aufweist. Die Spektralbereiche 24 gemäß der Fig. 7 sind zwischen 730 nm und 750 nm, beziehungsweise zwischen 790 nm und 810 nm. In diesen Spektralbereichen 24 kann etwa nach einem minimalen, respektive maximalem Intensitätswert gesucht werden, der mit einem Intensitätswert desselben Spektralbereichs 24 verglichen wird, der einem Verlauf bei einer anderen Medientemperatur entnommen wird.

Alternativ oder zusätzlich kann die Temperaturbestimmungseinheit 16 dazu eingerichtet sein, die Temperatur anhand wenigstens zweier unterschiedlicher spektraler Stützstellen 28 zu bestimmen. Wie in der schematischen Darstellung der Fig. 8 gezeigt, können die Stützstellen 28 derart gewählt werden, dass für sie das Absorptionsverhalten und/oder das Reflexionsverhalten des Mediums die gegenläufige Temperaturabhängigkeit aufweist. Die Stützstellen 28 können sich je nach Medium unterscheiden. Für das Medium, deren Intensitätswerte der Reflexion in der Fig. 8 schematisch dargestellt sind, liegen die Stützstellen 28 bei 740 nm und 800 nm. Durch eine Verhältnisbildung der Reflexion, bzw. der Intensitätswerte, bei den Stützstellen 28 bei 740 nm und 800 nm kann ein temperaturabhängiger Parameter berechnet werden, anhand dessen die Temperatur bestimmbar ist.

In der Fig. 9 ein ist eine Verschiebung eines Emissionspeaks eines Fluorophors eines anderen Mediums als jenes, dessen Reflexion in den Fig. 6 bis 8 dargestellt ist, schematisch dargestellt. Man erkennt, dass sich ein lokales Maximum zu einer höheren Wellenlänge verschiebt, wenn das Medium eine höhere Temperatur aufweist. Das Medium zeigt also ein temperaturabhängiges Reflexionsverhalten. Es versteht sich, dass die dargestellte Verschiebung exemplarisch und schematisch ist. Der Peak kann sich etwa für ein Medium in dem Wellenlängenbereich 22 zwischen 850 nm und 900 nm verschieben. Anhand des Medienparameters kann dies der Temperaturbestimmungseinheit 16 bekannt sein, weshalb gezielt in diesem Wellenlängenbereich 22 nach lokalen Maxima der Intensitätswerte des Emissionsspektrums gesucht wird. Anhand der Wellenlänge und/oder des Intensitätswerts der entsprechenden Maxima kann die Temperatur bestimmt werden.

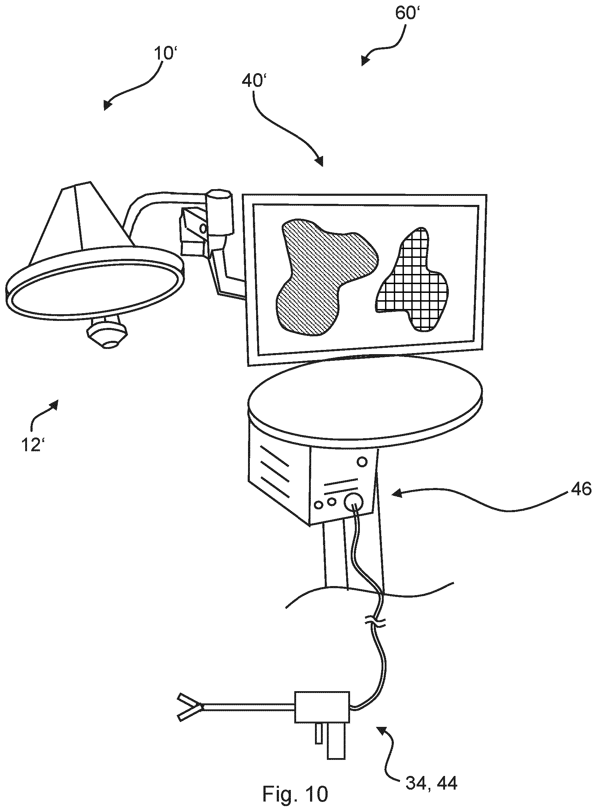

Fig. 10 zeigt eine schematische Darstellung einer weiteren Ausführungsform eines medizinischen Systems 60`. Das System 60' umfasst eine Bildgebungsvorrichtung 10', umfassend eine Bilderfassungseinheit 12', und eine Ausgabeeinheit 40' umfassend einen Bildschirm. Die Bildgebungsvorrichtung 10' ist als exoskopische Bildgebungsvorrichtung 10' ausgebildet. Das System 60' umfasst ebenfalls das Gerät 44, insbesondere das Behandlungsinstrument 34, das mittels der Geräteschnittstelle 46 an die Bildgebungsvorrichtung 10' angeschlossen ist. Eine Funktionsweise des Systems 60' kann der Funktionsweise des Systems 60 entsprechen. Im Unterschied werden Bilddaten eines Objektbereichs 14 erfasst, ohne dass die Bilderfassungseinheit 12' zumindest teilweise einer Kavität zugeführt wird.

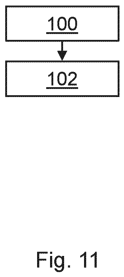

Fig. 11 zeigt ein schematisches Ablaufdiagramm eines Bildgebungsverfahrens durchgeführt mit der Bildgebungsvorrichtung 10, 10`. Das Verfahren umfasst einen Schritt 100 eines Erfassens von Bildern eines Objektbereichs 14 und Erzeugens multispektraler und/oder hyperspektraler Bilddaten des Objektbereichs 14, die räumliche und spektral Information umfassen. Ferner umfasst das Verfahren einen Schritt 102 eines Bestimmens einer Temperatur eines in dem Objektbereich 14 vorliegenden Mediums nach Maßgabe eines Medienparameters, der ein im Objektbereich 14 vorliegendes Medium anzeigt, und ferner nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht.

### Bezugszeichenliste

- 10: Bildgebungsvorrichtung
- 12: Bilderfassungseinheit
- 13: Endoskopvorrichtung
- 14: Objektbereich
- 15: Instrument
- 16: Temperaturbestimmungseinheit
- 18: Teilbereich
- 20: Teilbereich
- 22: Wellenlängenbereich
- 24: Spektralbereich
- 28: spektrale Stützstelle
- 30: Bildanalyseeinheit
- 32: Zielbereich
- 34: Behandlungsinstrument
- 36: Bereich
- 38: Umgebungsbereich
- 40: Ausgabeeinheit
- 41: Darstellung
- 42: Steuersignalerzeugungseinheit
- 44: Gerät
- 46: Geräteschnittstelle
- 60: medizinisches System
- 62: Bildgebungsgerät
- 66: Beleuchtungseinheit
- 68: Schaft
- 70: distaler Abschnitt
- 72: Recheneinheit
- 74: Fenster
- 76: distales Ende
- 78: proximaler Abschnitt
- 80: Handhabe
- 94: native Struktur
- 96: native Struktur
- 98: Gefäß
- 100: Schritt
- 102: Schritt

## Patentansprüche

1. Bildgebungsvorrichtung (10), insbesondere Endoskopvorrichtung (13), umfassend:
eine Bilderfassungseinheit (12), die dazu eingerichtet ist, multispektrale und/oder hyperspektrale Bilderfassung durchzuführen und Bilddaten eines Objektbereichs (14) zu erzeugen, die räumliche und spektral Information umfassen; und
eine Temperaturbestimmungseinheit (16), die dazu eingerichtet ist, nach Maßgabe eines Medienparameters, der ein im Objektbereich vorliegendes Medium anzeigt, und ferner nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht, eine Temperatur des Mediums zu bestimmen.

2. Bildgebungsvorrichtung (10) nach Anspruch 1,
wobei die Temperaturbestimmungseinheit (16) dazu eingerichtet ist, für unterschiedliche Teilbereiche (18) des Objektbereichs (14) jeweils eine Temperatur des Mediums zu bestimmen und/oder
wobei die Temperaturbestimmungseinheit (16) dazu eingerichtet ist, eine ortsaufgelöste Temperaturverteilung für wenigstens einen Teilbereich (20) des Objektbereichs (14) zu bestimmen.

3. Bildgebungsvorrichtung (10) nach einem der vorhergehenden Ansprüche,
wobei die spektrale Information Intensitätswerte umfasst, die sich auf einen Wellenlängenbereich (22) von, insbesondere vorzugsweise, 700 nm bis 1000 nm beziehen; und
wobei die Temperaturbestimmungseinheit (16) dazu eingerichtet ist, die Temperatur anhand der Intensitätswerte zu bestimmen, die sich auf den Wellenlängenbereich (22) von 700 nm bis 1000 nm beziehen.

4. Bildgebungsvorrichtung (10) nach Anspruch 3,
wobei die Temperaturbestimmungseinheit (16) dazu eingerichtet ist, die Temperatur anhand eines Abgleichs der spektralen Information mit einer bekannten Temperaturabhängigkeit eines Absorptionsspektrums, eines Reflexionsspektrums und/oder eines Fluoreszenzspektrums des Mediums zu bestimmen.

5. Bildgebungsvorrichtung (10) nach einem der vorhergehenden Ansprüche,
wobei die Temperaturbestimmungseinheit (16) dazu eingerichtet ist, zumindest zwei unterschiedliche Intensitätswerte miteinander zu vergleichen, die sich auf unterschiedliche Spektralbereiche (24) beziehen, um die Temperatur zu bestimmen.

6. Bildgebungsvorrichtung (10) nach einem der vorhergehenden Ansprüche,
wobei die Temperaturbestimmungseinheit (16) dazu eingerichtet ist, die Temperatur anhand wenigstens zweier unterschiedlicher spektraler Stützstellen (28) zu bestimmen, für die das Absorptionsverhalten, das Reflexionsverhalten und/oder das Fluoreszenzverhalten des Mediums eine gegenläufige Temperaturabhängigkeit aufweist.

7. Bildgebungsvorrichtung (10) nach einem der vorhergehenden Ansprüche,
wobei die Bilderfassungseinheit (12) dazu eingerichtet ist, in einem Referenzmodus und in einem Beobachtungsmodus betreibbar zu sein,
wobei in dem Referenzmodus zumindest ein Referenzbild des Objektbereichs (14) erfassbar ist, bevor dem Objektbereich (14) durch eine Behandlung Energie zugeführt wird, wobei das Referenzbild auf räumlich und spektral aufgelösten Bilddaten beruht;
wobei in dem Beobachtungsmodus zumindest ein Beobachtungsbild erfassbar ist, während dem Objektbereich (14) durch eine Behandlung Energie zugeführt wird, wobei das Beobachtungsbild auf räumlich und spektral aufgelösten Bilddaten beruht; und
wobei die Temperaturbestimmungseinheit (16) dazu eingerichtet ist, die Temperatur für das Beobachtungsbild nach Maßgabe des Referenzbilds und des Beobachtungsbilds zu bestimmen.

8. Bildgebungsvorrichtung (10) nach einem der vorhergehenden Ansprüche,
ferner umfassend eine Bildanalyseeinheit (30), die dazu eingerichtet ist, eine Bilderkennung durchzuführen und nach Maßgabe der Bilderkennung einen Zielbereich (32) des Objektbereichs (14) festzulegen und/oder über eine Bildsequenz zu verfolgen, wobei die Temperaturbestimmungseinheit (16) dazu eingerichtet ist, die Temperatur für den Zielbereich (32) zu bestimmen.

9. Bildgebungsvorrichtung (10) nach Anspruch 8,
wobei die Bilderkennung eine Erkennung eines Behandlungsinstruments (34) umfasst, und wobei bei dem Festlegen des Zielbereichs (32) ein Bereich (36) ausgespart wird, in dem sich das Behandlungsinstrument (34) und insbesondere zusätzlich ein definierter Umgebungsbereich (38) um das Behandlungsinstrument (34) herum befindet.

10. Bildgebungsvorrichtung (10) nach einem der vorhergehenden Ansprüche,
ferner umfassend eine Ausgabeeinheit (40), die dazu eingerichtet ist, dem Benutzer Information bereitzustellen, die sich auf die bestimmte Temperatur bezieht.

11. Bildgebungsvorrichtung (10) nach einem der vorhergehenden Ansprüche,
ferner umfassend:
eine Steuersignalerzeugungseinheit (42), die dazu eingerichtet ist, nach Maßgabe der bestimmten Temperatur ein Gerätesteuersignal für ein externes Gerät (44) wie beispielsweise ein Behandlungsinstrument (34) zu erzeugen; und
eine Geräteschnittstelle (46), an die ein externes Gerät (44) wie beispielsweise ein Behandlungsinstrument (34) anschließbar ist und die dazu eingerichtet ist, das Gerätesteuersignal auszugeben.

12. Medizinisches System (60),
umfassend:
eine Bildgebungsvorrichtung (10) nach Anspruch 11; und
ein Gerät (44), insbesondere ein Behandlungsinstrument (34), das an die Geräteschnittstelle (46) anschließbar ist und das dazu eingerichtet ist, das Gerätesteuersignal zu verarbeiten.

13. Bildgebungsverfahren, insbesondere durchgeführt mit einer Bildgebungsvorrichtung (10) nach einem der Ansprüche 1 bis 11, umfassend:
Erfassen von Bildern eines Objektbereichs (14) und Erzeugen multispektraler und/oder hyperspektraler Bilddaten des Objektbereichs (14), die räumliche und spektral Information umfassen; und
Bestimmen einer Temperatur eines in dem Objektbereich (14) vorliegenden Mediums nach Maßgabe eines Medienparameters, der ein im Objektbereich (14) vorliegendes Medium anzeigt, und ferner nach Maßgabe von in den Bilddaten enthaltener räumlicher und spektraler Information, die sich auf ein Absorptionsverhalten, ein Reflexionsverhalten und/oder ein Fluoreszenzverhalten des Mediums bezieht.

14. Bildgebungsverfahren nach Anspruch 13,
wobei das Medium eine medizinische Flüssigkeit, insbesondere eine medizinische Spüllösung, ist.

15. Bildgebungsverfahren nach Anspruch 13 oder 14,
wobei das Medium zumindest ein Additiv, insbesondere einen Farbstoff, enthält, das in einem Temperaturbereich zwischen 30°C und 100°C ein temperaturabhängiges Absorptionsspektrum, Reflexionsspektrum und/oder Fluoreszenzspektrum aufweist.
